# EUROPEAN PATENT SPECIFICATION

(11) **EP 0 948 320 B1**
(45) Date of publication and mention of the grant of the patent: **09.04.2003**
(21) Application number: 97944240.7
(22) Date of filing: 24.09.1997
(51) Int. Cl.: A61K 9/20, A61K 31/19

(54) **PHARMACEUTICAL COMPOSITIONS FOR SUSTAINED RELEASE OF THE HMG-CoA REDUCTASE INHIBITOR FLUVASTATIN**
ARZNEIMITTEL ZUR VERZÖGERTEN FREISETZUNG DES HMG-CoA REDUKTASE INHIBITORS FLUVASTATIN
COMPOSITIONS PHARMACEUTIQUES POUR LA LIBERATION PROLONGEE DE FLUVASTATINE INHIBANT LA REDUCTASE DE HMG-CoA

(30) Priority: 08.10.1996 SE 9603667
(43) Date of publication of application: 13.10.1999
(73) Proprietor: Novartis AG, 4056 Basel (CH); Novartis Pharma GmbH, 1230 Wien (AT)
(72) Inventor: LÖFROTH, Jan-Erik, S-431 69 Mölndal (SE); ÖDMAN, Jonas, S-412 62 Göteborg (SE)
(86) International application number: SE9701604
(87) International publication number: WO98015264

(56) References cited:
- EP-A- 0 465 096
- US-A- 5 356 896

## Description

### TECHNICAL FIELD

The present invention relates to sustained release pharmaceutical compositions comprising a water soluble salt of the HMG-CoA reductase inhibitor fluvastatin as active ingredient, said composition being selected from the group consitsing of matrix formulations, diffusion-controlled membrane coated formulations; and combinations thereof.

### BACKGROUND ART

### Sustained-release compositions

In recent years there has been a large increase in the development and use of sustained-release tablets which are designed to release the drug slowly after ingestion. With these types of dosage forms, the clinical utility of drugs can be improved by means of improved therapeutic effects, reduced incidence of adverse effects and simplified dosing regimens.

A sustained-release tablet releases the drug during several hours, typically more than 3 hours and less than 30 hours. Other commonly used terms such as "controlled release", "extended release", "prolonged release", etc., all comply with the definition of a product that releases the drug typically over more than 3 hours.

Several different types of formulations exist to obtain sustained release of a drug. The different formulations all aim to have release of the drug from the formulation, rather than the absorption process of the drug, as the rate limiting step. For this purpose, approaches based on the control of, e.g., dissolution, diffusion, swelling, osmotic pressure, complexation, ion-exchange, etc., can be employed. The actual approach taken for a given drug depends *inter alia* on the physical chemical properties of the drug. One of these is the solubility of the drug, which has a major impact on the pharmaceutical formulation strategy. A high solubility of the drug substance may induce problems, as discussed further below. However, in general, sustained release can be obtained according to the following principles, or combinations of them:
(i) By formulating the drug in an insoluble matrix. The gastrointestinal fluid penetrates the matrix, the drug is dissolved and diffuses out of the matrix and is absorbed. The driving force for diffusion is the concentration of the drug in the aqueous solution created by the penetrating gastrointestinal fluid. Thus, the higher the solubility, the higher the aqueous concentration of the drug in the matrix, and the faster the diffusional transport of the drug out of the matrix. If the matrix is a swelling matrix, e.g. a crosslinked (ionic) polymer with entrapped solid drug, the swelling kinetics of the matrix, the dissolution rate of the drug, and the diffusion of the drug will all contribute to the overall release rate. However, if the solubility of the drug is high, the release rate will be characterized by the diffusional transport after an initial swelling has occurred.
   A similar principle applies when drug particles or cores containing the active drug are coated with an insoluble but porous membrane of polymers. In this case, the gastrointestinal fluids penetrates the membrane, the drug is dissolved and thereafter diffuses out of the coated particle through the membrane. The driving force for diffusion is the concentration of the drug in the aqueous solution created by the penetrating gastrointestinal fluid. Thus, the higher the solubility, the higher the aqueous concentration of the drug in the matrix, and the faster the diffusional transport of the drug over the membrane. It can be argued that the transport rate with this type of formulation is dictated by the pores in the membrane. Nevertheless, it is the solubility which creates a high concentration gradient over the membrane and which then is important for the transport rate from the formulation.
(ii) By formulating the drug in an eroding matrix of, e.g. a soluble polymer. The rate with which the drug will be available at the absorption site is for these matrices a combination of the swelling and erosion rates of the matrix, and the dissolution and diffusion rates of the drug. A formulation based on this principle for a soluble drug might not show acceptable sustained release due to the high concentration gradient of the drug that can be created after an initial swelling of the polymer, leading to a diffusional transport of the drug instead of a release controlled by the erosion, i.e. the dissolution of the polymer.
(iii) Release controlled by osmotic pressure, whereby a semipermeable membrane is placed around a tablet or drug particle which allows transport of water into the formulation by osmosis. As a result of increased internal pressure when the drug dissolves, drug solution is then pumped out of the tablet through a small hole in the coating. The size of the orifice in the coating controls both the volume flow into the core reservoir, and the drug solution release rate. If the drug has a high solubility, the size of the orifice must be made small to prolong the release rate. This might then create problems with the possible build up of a high hydraulic pressure inside the device until the walls ruptures.

Improved drug delivery by sustained release has been discussed more extensively in the literature, e.g. in:
- Langer and Wise (Eds.) "Medical Applications of Controlled Release", vols I and II, CRC Press Inc, Boca Raton, 1984;
- Robinson and Lee (Eds.) "Controlled Drug Delivery - fundamentals and applications", Marcel Dekker, NY, 1987;
- Bogentoft and Sjögren, in "Towards Better Safety of Drugs and Pharmaceutical Products" (Ed: Breimer), Elsevier 1980.

EP-A-0465096 describes immediate or delayed release formulations of fluvistatin in tablet or capsule form.

US5356896 discloses sustained release tablet formulations of HMG-CoA inhibitors.

As mentioned above, the drug release from sustained release formulations is related to the drug solubility. The higher the water solubility of the drug, the faster the drug release and the shorter the duration of drug delivery. A fast release of the drug might mean that the desired rate and duration can not be obtained and that the beneficial effects of sustained release administration are lost. Thus, a special challenge is met when trying to formulate water soluble substances for sustained release formulations. One way to try to solve this problem would be to include large amounts of slow release exipients in the formulation. However, this approach has drawbacks such as increased costs and increased size of the formulation. Increased physical size of the dosage form may present problems for some patients, since the tablet will be more difficult to swallow. Another possibility is to use a less water soluble salt. However, such a change requires a more extensive development work and may also lead to bioavailability problems due to incomplete dissolution.

### HMG-CoA Reductase Inhibitors

Hypercholesterolemia is related to an increased risk of coronary heart diseases. A possible way to reduce cholesterol levels in a patient is to inhibit the enzyme 3-hydroxy-3-methyl-glutaryl coenzyme A (HMG-CoA) reductase, which is a key enzyme in the regulation of cholesterol biosynthesis. The HMG-CoA reductase inhibitors constitute a well known group of therapeutic agents for the treatment of hypercholesterolemia, which group comprises fermentation products such as lovastatin and pravastatin, as well as semi-synthetic analogs such as simvastatin. More recently have completely synthetic drugs, e.g. fluvastatin, been developed.

The use of some HMG-CoA reductase inhibitors for the preparation of a medicament adapted for time-controlled administration is disclosed in EP-B-0 375 156.

Fluvastatin (R*, S*-(E)-(±)-7-[3-(4-fluorophenyl)-1-(1-methyl-ethyl)-1H-indol-2-yl]-3,5-dihydroxy-6-heptenoic acid) is known from EP-A-0 114 027.

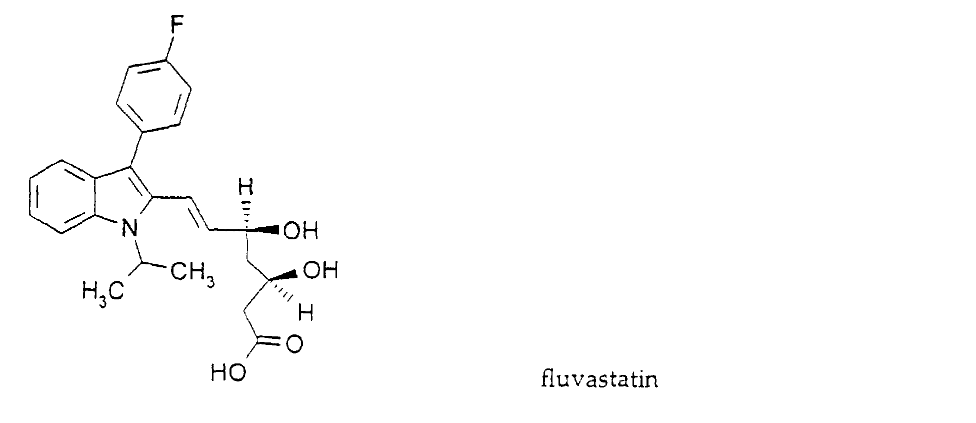

Fluvastatin is a water soluble drug. For example, the solubility of the sodium salt of fluvastatin in water extends to more than 50 g/l. Biopharmaceutical requirements of a sustained release product of this water soluble drug would then at first sight impose formulation problems, as discussed above. Thus, with a diffusion controlled release device for this soluble substance, e.g. an insoluble matrix of a polymer, fast release rates can be expected due to the high solubility of fluvastatin creating high concentration gradients as the driving force for diffusion out of the matrix.

Second, an eroding matrix of fluvastatin is not expected to be useful due to the high concentrations of the drug in solution that can be the result when the gastrointestinal fluid penetrates the matrix. The erosion of the matrix, e.g. by dissolution of the outer hydrated polymer layers, would then indeed not be a rate controlling factor, except perhaps only for a first initial short time during hydration and swelling of the matrix.

Finally, advanced techniques with high production costs are expected to be necessary to produce osmotic pressure controlled formulations. The high solubility of fluvastatin is also expected to complicate the action of such formulations. Thus, a srnall orifice would be needed in order to keep the rate low with which the amount of drug is pumped out through such devices. With a small orifice, however, the hydrostatic pressure that will be built up would put demands on the choice of a strong polymer membrane.

Consequently, there is a need for pharmaceutical formulations of HMG-CoA reductase inhibitors which avoid the above mentioned drawbacks and are possible to prepare, e.g., without including large amounts of slow release excipients or the use of highly advanced techniques. Preferably, the production costs of the formulations should be low.

### BRIEF DESCRIPTION OF THE DRAWINGS

Fig. 1: Release of fluvastatin and methylparaben, and tablet erosion, from sustained-release tablets based on polyethylene oxide (PEO) 8,000,000.
Fig. 2: Release of fluvastatin, methylparaben and diclofenac from sustained-release tablets based on xanthane.
Fig. 3: Release of fluvastatin and methylparaben from sustained-release tablets based on paraffin, and release of fluvastatin from immediate release (IR) capsules.
Fig. 4: Release rate of fluvastatin and diclofenac over a polymeric membrane in a two-compartment cell at different concentrations in donor chamber.

### DISCLOSURE OF THE INVENTION

It has surprisingly been found that sustained-release compositions, comprising fluvastatin as a water soluble salt, exhibit particularly favorable release characteristics such as unexpectedly long duration and slow rate of drug release. In the present context, the term "water soluble" should be understood as a solubility of more than 30 mg/ml in water at +37°C.

Consequently, the present invention provides a pharmaceutical composition for sustained release comprising a water soluble salt, preferably the sodium salt, of fluvastatin as an active ingredient. The sustained-release fluvastatin compositions for which these favorable properties are obtained are selected from the group comprising matrix formulations, diffusion-controlled membrane coated formulations; and combinations thereof.

The said eroding and non-eroding matrix formulations can be based on hydrophilic and/or hydrophobic matrix forming excipients. The matrix and membrane coated formulations may be monolithic, such as tablets, or in the form of multiple units administered in a tablet, capsule or sachets.

The hydrophilic or hydrophobic, eroding or non-eroding, matrix material and the material for film formation, can be:
- cellulose derivatives such as ethyl cellulose, hydroxypropyl methyl cellulose, hydroxypropyl cellulose, ethyl hydroxyethyl cellulose, carboxymethyl cellulose, cellulose acetate butyrate, cellulose acetate phtalate, etc;
- polysaccharides, like alginate; xanthane; carrageenan; scleroglucan; pullulan; dextran; haluronic acid; chitin; chitosan; starch; etc;
- other natural polymers, like proteins (e.g albumin, gelatine); natural rubber; etc;
- synthetic polymers, like acrylates (e.g. polymethacrylate, poly(hydroxy ethyl methacrylate), poly(methyl methacrylate), poly(hydroxy ethyl methacrylate - co methyl methacrylate), Carbopol 934™); polyamides (e.g. polyacrylamide, poly(methylene bisacrylamide)); polyanhydrides (e.g. poly(bis carboxyphenoxy)methane); PEO-PPO block-co-polymers (e.g. poloxamers, etc); polyvinyl chloride; polyvinyl pyrrolidone; polyvinyl acetate; polyvinyl alcohol; polyethylene, polyethylene glycols and co-polymers thereof; polyethylene oxides and co-polymers thereof; polypropylene and co-polymers thereof; polystyrene; polyesters (e.g. poly(lactic acid), poly(glycolic acid), poly(caprolactone), etc, and co-polymers thereof, and poly(ortho esters), and co-polymers thereof): resins (e.g. Dowex™, Amberlite™); polycarbonate; cellophane; silicones (e.g. poly (dimethylsiloxane)); polyurethanes; synthetic rubbers (e.g. styrene butadiene rubber, isopropene rubber); etc;
- others, like shellacs; waxes (e.g. carnauba wax, beeswax, glycowax, castor wax); nylon; stearates (e.g. glycerol palmitostearate, glyceryl monostearate, glyceryl tristearate, stearyl alcohol); lipids (e.g. glycerides, phospholipids); paraffin; etc.

Combinations of the above mentioned materials are also possible.

In a preferred form, the invention provides a pharmaceutical composition as described above which is an eroding matrix formulation, wherein the matrix material is selected from the group comprising polyethylene oxide, hydroxypropyl methyl cellulose and paraffin.

In another preferred form, the said pharmaceutical composition is a non-eroding matrix formulation, wherein the matrix material is selected from the group comprising xanthane and polyvinylchloride.

In yet another preferred form, the said pharmaceutical composition is a diffusion-controlled membrane coated formulation, wherein the material for film formation is selected from the group comprising ethyl cellulose, hydroxypropyl methyl cellulose and hydoxypropyl cellulose.

In the present context, the term "fluvastatin" comprises both of the pure enantiomers, as well as racemic mixtures.

The water soluble salts of fluvastatin to be used in the compositions according to the invention comprise e.g. the sodium, potassium, ammonium salts. The sodium salt is preferred.

The pharmaceutical formulations according to the invention are useful for lowering the blood cholesterol level in animals, in particular mammals, e.g. humans. They are therefore useful as hypercholesterolemic and anti-atherosclerotic agents.

Consequently, the invention provides in another aspect the use of fluvastatin for the manufacture of a pharmaceutical composition for sustained release, for the treatment of hypercholesterolemia. Preferably, the said composition is selected from the group comprising matrix formulations, diffusion-controlled membrane coated formulations; and combinations thereof.

In yet another aspect, the invention provides a method for the treatment of hypercholesterolemia comprising administering to a mammal, including man, a therapeutically effective amount of a pharmaceutical composition for sustained release, comprising fluvastatin. Preferably, the said composition is selected from the group comprising matrix formulations, diffusion-controlled membrane coated formulations; and combinations thereof.

The pharmaceutical formulations according to the invention can be prepared by use of well known pharmaceutical processing techniques such as blending, granulation, milling, spray drying, compaction, or coating.

The typical daily dose of the active substance fluvastatin varies within a wide range and will depend on various factors such as for example the individual requirement of each patient and the disease. In general, sustained-release dosages will be in the range of 1 to 1000 mg of fluvastatin per day, preferably 2 to 200 mg/day.

### EXAMPLES OF THE INVENTION

To exemplify the unexpectedly favorable properties of fluvastatin in matrix formulations and membrane coated formulations, the release profile of fluvastatin sodium (water solubility > 50 mg/ml) was compared with two other water soluble drugs, namely methylparaben (methyl p-hydroxybenzoate; water solubility ≈ 2 mg/ml) and diclofenac sodium (2-[(2,6-dichlorophenyl)amino] benzeneacetic acid monosodium salt; water solubility ≈ 5 mg/ml).

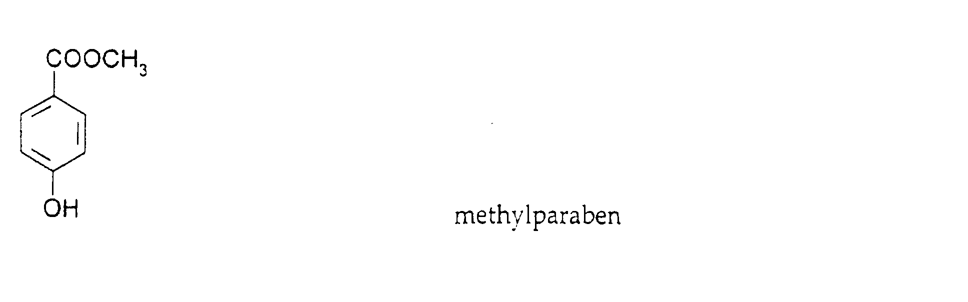

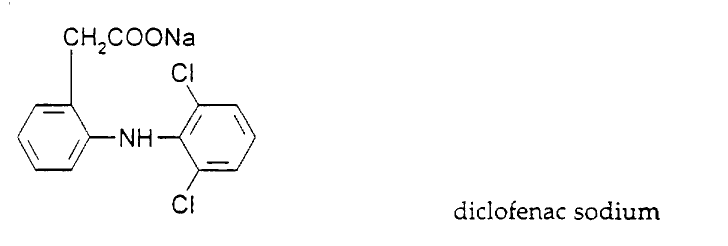

Methylparaben and diclofenac sodium could be expected to exhibit a somewhat slower release rate and longer duration of release, due to the lower water solubility. However, unexpectedly, the release of fluvastatin was consistently slower than methylparaben and diclofenac sodium for all the tested types of sustained-release formulations.

In the following Examples 1 to 3, drug release from various types of tablets was determined in pH 6.8, +37°C, by use of an USP II apparatus at a paddle stirring rate of 75 rpm. All tablet formulations were manufactured by conventional techniques and, for each example, in an identical manner except for the drug constituent.

### EXAMPLE 1: Drug release and tablet erosion for eroding polyethyleneoxide (PEO) matrix tablet.

Fluvastatin or methylparaben (10 mg each) were formulated in an eroding matrix of PEO 8,000,000 (58 mg) and magnesium stearate (0.7 mg). Tablet erosion was determined by weighing after removal of the tablets from the dissolution apparatus and drying to constant weight.

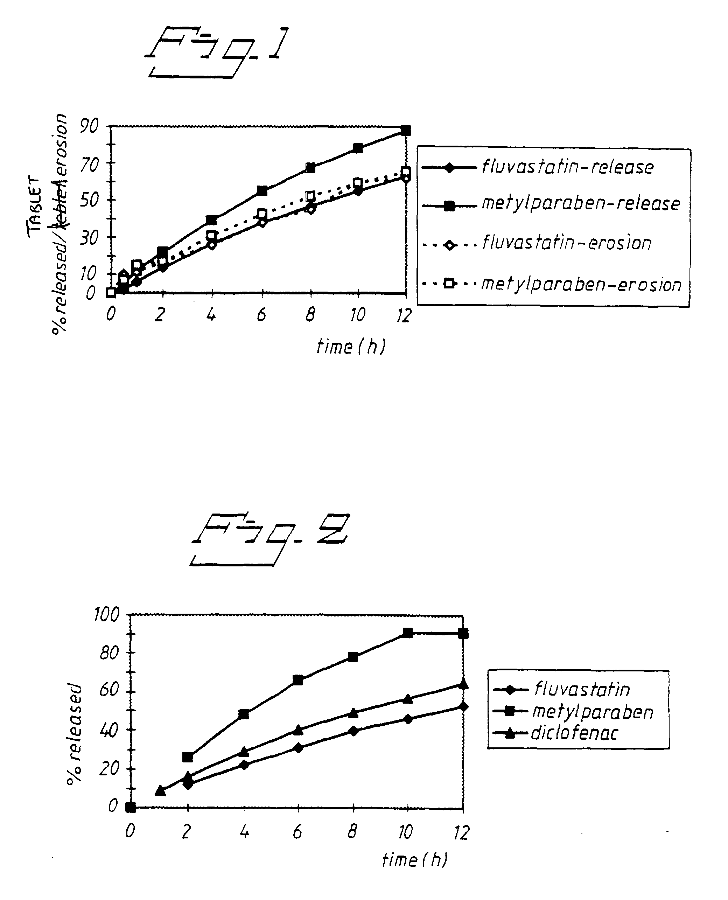

The results (Fig. 1) show that release of fluvastatin from the sustained release tablet was slower than the release of methylparaben in spite of the higher solubility. The tablet erosion and drug release was almost identical for fluvastatin whereas for the methylparaben tablet, as could be expected for a water soluble drug, the drug release was faster than the tablet erosion. This was a further indication that fluvastatin has unexpectedly favourable extended release properties when administered as an eroding matrix tablet both compared to what could expected from tablet erosion data and compared to another somewhat less soluble drug.

### EXAMPLE 2: Release from a non eroding high molecular weight xanthane matrix tablet.

Fluvastatin, methylparaben or diclofenac (5 mg each) were formulated in a non-eroding matrix of xanthane (195 mg).

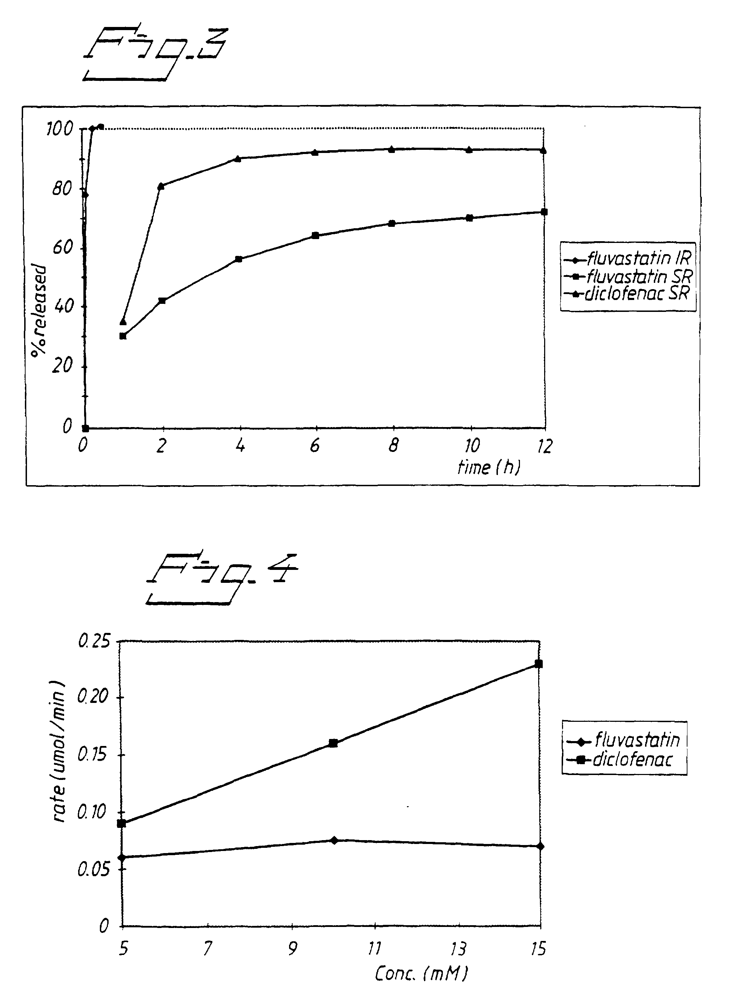

The results (Fig. 2) show that release of fluvastatin from the sustained release tablet was slower than the release of both diclofenac and methylparaben despite the higher solubility. This provides an example that fluvastatin has unexpectedly favourable extended release properties when administered as a non-eroding matrix tablet.

### EXAMPLE 3: Release from eroding paraffin matrix tablet and from a conventional (immediate release) hard gelatin capsule

Fluvastatin or diclofenac (20 mg each) were formulated in an eroding matrix of paraffin (120 mg), lactose (30 mg), ethyl cellulose (3 mg) and magnesium stearate (1.7 mg). The immediate release capsule was a hard gelatine capsule containing 20 mg of fluvastatin.

The results (Fig. 3) show that release of fluvastatin from the sustained release tablet was slower than the release of diclofenac despite the higher solubility. This provides another example that fluvastatin has unexpectedly favorable extended release properties when administered as a matrix tablet.

The drug release for the immediate release capsule was almost immediate in contrast to the duration of drug release of more than 10 hours for fluvastatin sustained-release. This result indicates that the unexpectedly slow release for fluvastatin is not a general property for all kinds of oral fluvastatin formulations, but is limited to certain types of sustained release formulations according to the invention.

### EXAMPLE 4: Transport over a diffusion controlling membrane.

The release rate of fluvastatin sodium and diclofenac sodium was studied over a polymeric membrane from a donor compartment initially containing all drug substance, thus corresponding to a membrane coated formulation containing the active drug, to a receiving chamber which simulated the medium where the drug is released. The release rate was studied at different initial concentrations of the active drug. The solutions (pH 6.8) in the chambers were well stirred and thermostated at +37°C. From the cumulative amount released versus time, the release rates (amount released/time) were determined as the slopes of the linear parts of the curves obtained at steady state. No accumulation in the membrane was found of any of the drugs. The results are presented in Fig. 4 as the release rates versus the concentrations used in the experiments.

The release rate of diclofenac increased as expected when the concentration of diclofenac was increased in the donor chamber. However, surprisingly the release rate of fluvastatin was independent of the concentration of fluvastatin in the donor compartment, resulting in a release rate of fluvastatin that was much slower compared to diclofenac. This strengthens that an unexpectedly slow release rate can be maintained for fluvastatin in such formulations irrespective of the amount of dissolved drug within a membrane coated formulations.

### EXAMPLE 5: Manufacture of pharmaceutical formulations

### 5.1.

A dosage form adapted, designed and shaped for the oral delivery of fluvastatin sodium to a patient in need of fluvastatin therapy is manufactured as follows: first 30.0 g of fluvastatin sodium, 90.0 g of paraffin, 50.0 g calcium carbonate and 20.0 g sorbitol are screened through a 1.0 mm screen. The screened material are mixed in a planetary mixer for 10 minutes to produce a homogenous blend. Then, a granulation solution is prepared by dissolving 2.0 g ethyl cellulose (10 cps) in 150.0 g 95% ethanol during constant stirring for 6 hours. The granulation solution is slowly added to the dry mixture during agitation, to yield a wet granulation. The granulation is dried at +50°C for 12 hours. After drying, the granulation is passed through a screen of 1.5 mm. Magnesium stearate (2.0 g) is mixed in to the granulate for 3 minutes. Then, 8 mm round tablets, each comprising 30 mg of fluvastatin sodium are compressed in a Korsch® press under a pressure of 25 kN.

### 5.2.

Fluvastatin sodium (20.0 g), 150.0 g of hydroxypropyl methyl cellulose (molecular weight 30,000), 30.0 g of sorbitol, 30.0 g of sodium aluminium silicate are dry mixed in a planetary mixer for 5 minutes. Then, a granulation solution is prepared by dissolving 10.0 g of polyvinyl pyrrolidone (molecular weight 360,000) in 200 g of 99.5% ethanol. The granulation solution is slowly added to the dry mixture during agitation, to yield a wetted mass. The granulation is dried overnight at +60°C. Next, the granulation is milled in a oscillating granulator through a screen of 0.7 mm. Magnesium stearate (2.0 g) is mixed with the granulation for 2 minutes. Then, extended release round 10 mm tablets are prepared by compressing the composition with a 30 kN compression force. This fluvastatin tablet comprises 20 mg of fluvastatin sodium.

### 5.3.

Fluvastatin sodium (10 g), 50 g of 8,000,000 molecular weight polyethylene oxide, 50 g lactose are dry mixed. Then, 60 g of 99.5% ethanol and the dry mixture are slowly mixed together in a planetary mixer for 5 minutes. The granulate is dried for 12 hours in +45°C. Next, the granulation is passed through a 1.0 mm screen. 1.0 g of magnesium stearate is mixed with the granulation for 2 minutes. Then, extended release round 8 mm tablets are prepared by compressing with a 20 kN compression force. This fluvastatin tablet comprises 10 mg of fluvastatin sodium.

### 5.4.

Fluvastatin tablets is manufactured as follows: first, 3 g of fluvastatin sodium, 20 g of 30,000 molecular weight hydroxypropyl methyl cellulose, 10 g of sodium aluminium silicate and 0.2 g carboxypolymethylene are dry mixed. Then, a granulation solution is prepared by dissolving 2.0 g ethyl cellulose (10 cps) in 20.0 g 99.5% ethanol. The granulation solution is slowly added to the dry mixture during agitation, to yield a wet granulation. The granulate is dried for 12 hours in 45°C. Next, the granulation is passed through a 1.0 mm screen. Sodium stearyl fumarate (0.8 g) is mixed with the granulation for 2 minutes. Then, extended release round 11 mm tablets are prepared by compressing with a 25 kN compression force. This fluvastatin tablet comprises 20 mg of fluvastatin sodium.

### 5.5.

After the initial forming of beads containing fluvastatin sodium, the beads obtained are coated with the polymeric layer controlling the release from the pellet, example of this coating is described below. The polymeric mixture is dissolved in an organic solvent such as ethanol, isopropyl alcohol and/or methylene chloride. The spraying can be carried out in a coating pan, but is preferably carried out in a fluidized bed.

| | |
|---|---|
| Fluvastatin sodium | 300 g |
| Methylene chloride | 2000 g |
| Ethanol 99.5% | 1000 g |
| SiO₂ (0.15-0.25) | 100 g |

| Polymeric layer | |
|---|---|
| Ethyl cellulose 10 cps | 65.0 g |
| Hydroxypropyl methyl cellulose | 15.0 g |
| Acetyltributyl citrate | 9.0 g |
| Methylene chloride | 1500 g |
| Isopropylic alcohol | 350 g |

A solution is prepared by dissolving fluvastatin sodium in 99.5% ethanol and methylene chloride, the solution is then sprayed onto the cores of silicon dioxide in a fluidized bed. 100 g of the beads (fraction 0.4-0.65 mm) are covered with the polymeric layer contairing ethyl cellulose 10 cps, hydroxypropyl methyl cellulose and acetyltributyl citrate by spraying a solution of the mentioned substances in methylene chloride and isopropylic alcohol. The coated beads are then filled into hard gelatin capsules.

## Claims

1. A sustained release pharmaceutical composition comprising a water soluble salt of fluvastatin as active ingredient and being selected from the group consisting of matrix formulations, diffusion-controlled membrane coated formulations and combinations thereof.

2. A pharmaceutical composition according to claim 1 wherein the said water soluble salt of fluvastatin is the sodium salt.

3. A pharmaceutical composition according to claim 1 or 2 which is an eroding matrix formulation.

4. A pharmaceutical composition according to claim 3 wherein the matrix material is selected from the group consisting of polyethylene oxide, hydroxypropyl methyl cellulose and paraffin.

5. A pharmaceutical composition according to claim 1 or 2 which is a non-eroding matrix formulation.

6. A pharmaceutical composition according to claim 5 wherein the matrix material is selected from the group comprising xanthane and polyvinylchloride.

7. A pharmaceutical composition according to claim 1 or 2 which is a diffusion-controlled membrane coated formulation.

8. A pharmaceutical composition according to claim 7 wherein the material for film formation is selected from the group consisting of ethyl cellulose, hydroxypropyl methyl cellulose and hydoxypropyl cellulose.

9. A pharmaceutical composition according to any one of claims 1 to 8 for use in the treatment of hypercholesterolemia.

10. The use of a water soluble salt of fluvastatin for the manufacture of a sustained release pharmaceutical composition for the treatment of hypercholesterolemia.

11. The use according to claim 10 wherein the said pharmaceutical composition is selected from the group consisting of matrix formulations, diffusion-controlled membrane coated formulations; and combinations thereof.

## Patentansprüche

1. Verzögert freisetzende Zusammensetzung, die ein wasserlösliches Fluvastatinsalz als Wirkstoff enthält und aus der Gruppe ausgewählt ist. die besteht aus Matrixformulierungen, diffusionskontrollierten, membranbeschichteten Formulierungen und Kombinationen hiervon.

2. Pharmazeutische Zusammensetzung nach Anspruch 1, worin das wasserlösliche Fluvastatinsalz das Natriumsalz ist.

3. Pharmazeutische Zusammensetzung nach Anspruch 1 oder 2, die eine erodierende Matrixformulierung ist.

4. Pharmazeutische Zusammensetzung nach Anspruch 3, worin das Matrixmaterial aus der Gruppe ausgewählt ist, die besteht aus Polyethylenoxid. Hydroypropylmethylcellulose und Paraffin.

5. Pharmazeutische Zusammensetzung nach Anspruch 1 oder 2, die eine nicht-erodierende Matrixformulierung ist.

6. Pharmazeutische Zusammensetzung nach Anspruch 5, worin das Matrixmaterial aus der Gruppe ausgewählt ist. die Xanthan und Polyvinylchlorid umfaßt.

7. Pharmazeutische Zusammensetzung nach Anspruch 1 oder 2, die eine diffusionskontrollierte, membranbeschichtete Formulierung ist.

8. Pharmazeutische Zusammensetzung nach Anspruch 7, worin das Material für die Filmbildung aus der Gruppe ausgewählt ist, die besteht aus Ethylcellulose. Hydroxypropylmethylcellulose und Hydroxypropylcellulose.

9. Pharmazeutische Zusammensetzung nach einem der Ansprüche 1 bis 8 zur Verwendung bei der Behandlung von Hypercholesterinämic.

10. Verwendung eines wasserlöslichen Fluvastalinsalzes zur Herstellung einer anhaltend freisetzenden pharmazeutischen Zusammensetzung zur Behandlung der Hypercholesterinämie.

11. Verwendung nach Anspruch 10, worin die pharmazeutische Zusammensetzung aus der Gruppe ausgewählt ist. die besteht aus Matrixformulierungen, diffusionskontrollierten, membranbeschichteten Formulierungen und Kombinationen hiervon.

## Revendications

1. Composition pharmaceutique à libération prolongée comprenant un sel de fluvastatine hydrosoluble comme ingrédient actif, et qui est choisie dans le groupe constitué par des formulations de matrice, des formulations revêtues d'une membrane à diffusion contrôlée et leurs combinaisons.

2. Composition pharmaceutique suivant la revendication 1, dans laquelle ledit sel de fluvastatine hydrosoluble est le sel de sodium.

3. Composition pharmaceutique suivant la revendication 1 ou 2, qui est une formulation à matrice érodante.

4. Composition pharmaceutique suivant la revendication 3, dans laquelle le matériau de la matrice est choisi dans le groupe constitué par l'oxyde de polyéthylène, l'hydroxypropylméthylcellulose et la paraffine.

5. Composition pharmaceutique suivant la revendication 1 ou 2, qui est une formulation de matrice non érodante.

6. Composition pharmaceutique suivant la revendication 5, dans laquelle le matériau de matrice est choisi dans le groupe constitué par le xanthane et le chlorure de polyvinyle.

7. Composition pharmaceutique suivant la revendication 1 ou 2, qui est une formulation revêtue d'une membrane à diffusion contrôlée.

8. Composition pharmaceutique suivant la revendication 7, dans laquelle le matériau de formation de film est choisi dans le groupe constitué par l'éthylcellulose, l'hydroxypropylméthylcellulose et l'hydroxypropyl-cellulose.

9. Composition pharmaceutique suivant l'une quelconque des revendications 1 à 8, pour une utilisation dans le traitement de l'hypercholestérolémie.

10. Utilisation d'un sel de fluvastatine hydrosoluble pour la fabrication d'une composition pharmaceutique à libération prolongée pour le traitement de l'hypercholestérolémie.

11. Utilisation suivant la revendication 10, dans laquelle ladite composition pharmaceutique est choisie dans le groupe constitué par des formulations de matrice, des formulations revêtues d'une membrane à diffusion contrôlée ; et leurs combinaisons.
